# EUROPEAN PATENT APPLICATION

(11) **EP 1 604 649 A1**
(43) Date of publication of application: **14.12.2005**
(21) Application number: 04013667.3
(22) Date of filing: 09.06.2004
(51) Int. Cl.: A61K 9/00

(54) **Composite material for use as protein carrier**

(71) Applicant: Scil Technology GmbH, 82152 Martinsried (DE)
(72) Inventor: Schütz, Andreas, 82152 Krailling (DE); Siedler, Michael, 80538 München (DE); Hellerbrand, Klaus, 82269 Geltendorf (DE)
(74) Representative: Grünecker, Kinkeldey, Stockmair & Schwanhäusser Anwaltssozietät

(57) **Abstract**

The present invention relates to a material having osteoinductive and osteoconductive properties in vivo comprising a ceramic carrier, preferably containing calcium phosphate, and an active agent, preferably an osteoinductive protein / peptide or a drug, and a polymer, wherein the active agent is homogeneously coated on the carrier and within the polymer, which is preferably a degradable polymer.

Said polymer modulates the release kinetic of the active agent and protects same from degradation to prolong the half-life in vivo. Moreover, the present invention relates to a method for the production of a material having osteoinductive and osteoconductive properties in vivo. The invention encompasses a pharmaceutical composition comprising the material of the invention or a material which is obtainable by the method of the invention and relates to the use of said material for the preparation of a pharmaceutical composition for tissue regeneration, especially bone augmentation or treatment of bone defects, for treating degenerative and traumatic disc disease and for treatment of bone dehiscence. Finally, the invention relates to a kit comprising the material of the invention or a material, which is obtainable by the method of the invention.

## Description

The present invention relates to a material having osteoinductive and osteoconductive properties in vivo comprising a ceramic carrier, preferably containing calcium phosphate, and an active agent, preferably an osteoinductive protein / peptide or a drug, and a polymer, wherein the active agent is homogeneously coated on the carrier and within the polymer, which is preferably a degradable polymer.

Said polymer modulates the release kinetic of the active agent and protects same from degradation to prolong the half-life in vivo. Moreover, the present invention relates to a method for the production of a material having osteoinductive and osteoconductive properties in vivo. The invention encompasses a pharmaceutical composition comprising the material of the invention or a material which is obtainable by the method of the invention and relates to the use of said material for the preparation of a pharmaceutical composition for tissue regeneration, especially bone augmentation or treatment of bone defects, for treating degenerative and traumatic disc disease and for treatment of bone dehiscence. Finally, the invention relates to a kit comprising the material of the invention or a material, which is obtainable by the method of the invention.

### Background Technology

Various calcium phosphates such as beta-tricalcium phosphate (Ca₃(PO₄)₂) (beta-TCP), alpha-tricalcium phosphate (alpha-TCP) and hydroxyapatite (HA) have been shown to be effective as bone replacement materials. Beta-TCP, for example, is suitable both as granulate and in pieces (blocks) for the treatment of bone defects. The bone replacements materials containing calcium phosphate are usually used when the regeneration of the bone is not possible any more or is possible with difficulties only. In addition, bone replacement materials are used when the formation of additional bone is a prerequisite for a subsequent setting of an implant. The calcium phosphates exhibit an osteoconductive effect, i.e. they represent an inert structure facilitating the migration of cells from the neighbouring bone. The presence of bones or different mesenchymal cells, however, is a precondition for the new formation of bones.

The effect of calcium phosphates can be significantly increased by adding bone chips. The bones are not only osteoconductive, but also osteoinductive, i.e. they cause the transformation of undifferentiated mesenchymal stem cells into osteoblasts and chondrocytes. For reasons of safety, autogenic bone chips are preferred to the allogenic or xenogenic preparations. The production of autogenic bones, however, always involves a second surgical procedure, which, in many cases, is not accepted by the patient.

Another important biomaterial class which plays a predominant role in the field of bone tissue engineering are bioresorbable polymers (Vert, 1989). Especially the compound class of poly(hydroxy acids) has interesting application prospects due to their intrinsic biodegradability. These materials of which poly(glycolic acid) (PGA) and poly(lactic acid) (PLA) are the most prominent undergo hydrolytic chain cleavage (degradation) in a moist environment. Sustained degradation finally leads to the corresponding hydroxy acid units. Most of these hydrolytic end products occur as metabolites of many bacteria and cell phenotypes.

The degradation potential and their mechanical properties offers application for the use as substrates for temporary implants in medical technology. Studies for various polymers in different tissues document the excellent biocompatibility of these compounds in vivo forming the bases for the development of commercial implants as medical devices (Middleton, 2000).

In clinical surgery, polyesters presumably play the most important role in connection with the fixation, augmentation and replacement of bone. Devices like screws, plates, anchors or pins serve for positioning and fixation of bone fragments after bone loss or damage. The major feature of these absorbable polymers in application is the lacking necessity for a removal operation. Another important point is in favor of polymeric fixation devices: the mechanical integration of the implant in the bone tissue.

An important drawback in totally polyester based applications is the possible accumulation of degradation products reaching cytotoxic levels and the accompanying acidification at the implant site due to the pH lowering release of acid monomers.

To avoid such negative consequences caused by local pH decrease it has been suggested to incorporate basic salts within PLA / PGA implants (Agrawal et al., 1997). Other approaches employ basic ceramics like calcium phosphates as fillers for polyesters to balance the local pH value and increase the mechanical stability (Schiller 2003). Also composites of hydroxyapatite and PLA/PLGA (polylactide-co-glycolide) have been produced (Durucan 2000). The processing of such composites requires thermal treatment and the use of chloroform solutions of the polymeric component. As an alternative one can use polymers with higher hydrolytic stability like poly(hydroxybutyric acid).

For the use of these polymers as bone substitutes the common strategy is to design a implant which temporarily fulfills the function to allow a healing process and to retain strength during the early stages at the implantation site after operation. Afterwards the loss of strength and modulus of the implant should be in harmony with the increasing strength of the injured tissue (Tormälä, 1995). Proceeding degradation creates space for restoring processes to fill the gap with ingrown of vital host tissue. Presently, no filling material is available that fits this requirements satisfactorily to form new homogeneous bone in large defects (Rueger 1996).

To achieve an osteoinductive effect an alternative to the use of autogenic bones is the use of specific bone growth and differentiation factors such as GDF-5 or different bone morphogenetic proteins (BMPs). The osteoinductive effect, however, can only be exerted by these protein factors if they are used in an immobilized form. In the literature, calcium phosphates, collagen, mineralised collagen (collagen-containing calcium phosphate) and bioresorbable polymers are described as carriers (hydroxy apatite and beta-TCP (Hotz, 1994), hydroxylic apatite from algae extracts (Gao, 1996), bone extracts (Gombotz, 1996), collagen (Friess, 1999) and poly(α-hydroxy acids) (Hollinger, 1996).

The analyses of the potency of the coated carriers, which are described in the literature, do not present a uniform picture but exhibit significant variations which are a consequence of either the carrier type selected or the coating method (Terheyden et al. (1997)). Various methods are described.

In WO 98/21972 coating by rapid precipitation of GDF-5 onto beta-TCP is achieved by first dissolving GDF-5 in an organic solvent and then precipitating it by adding water. Due to the toxicity of many solvents, however, such a process is not preferred for the production of pharmaceutical compositions. Lind et al. (1996) carry out the coating of various calcium phosphate ceramics in the presence of gelatine (usually obtained from bovine or pig bones) as protection protein. Due to the increased risk of infection and immunogenic reactions, however, the use of animal substances should be avoided for the production of pharmaceutical compositions and medicinal products. Friess et al. (1999) and Gao et al. (1996) describe the coating of collagens with BMP-2. Due to the low compressive strength of collagens, such carriers, however, are not suitable for many indications. This particularly applies to indications with which the newly-formed bone has to sustain a later pressure load. Furthermore, pharmaceutical qualities of collagen are so far available from animal sources only. Finally, according to the fast degradation rate and release of the growth factors in the state of the art products (e.g. rhBMP-2 and collagen sponge) the drug substance content is often dramaticaly above the physiological level in the bone tissue.

Advantageously, as disclosed in WO 03/043673, it has been found by the present inventors that improved and reliable osteoinductive and osteoconductive properties in vivo after implantation into a subject, preferably a human, is achieved in a device, wherein a homogenous distribution of the composite carrier, such as β-TCP or other calcium phosphates, with biologically active, non-aggregated osteoinductive protein can be realized. Such aggregation causes micro-precipitation, which is the reason for an inhomogenous distribution resulting in at least significantly decreased osteoinductive properties as described for other devices in the prior art, e.g., in WO98/21972. Moreover, it has been found that undesirable side effects, such as inflammation and toxic reactions of the subject after implantation, can be avoided by the material of the present invention which is free of toxic impurities or infectious contaminants. In particular, the use of protecting proteins (such as e.g. gelatine) as solubility mediator is totally unnecessary for the device of WO 03/043673. However, such devices are not suitable for applications requiring a retarded release of the active agent.

In the field of bone augmentation retarded release systems are especially required in view of short half-life of proteins or peptides in the human body with respect to bone induction, either due to dispersion from the implant site or through degradation. In first attempts to achieve a retarded release of bone morphogenic proteins, devices have been disclosed, wherein such proteins have been combined with bioresorbable polymers. Hollinger et al (1996) published the use of poly (α-hydroxy acids) as carriers for BMP-2. In combination with osteogenic proteins or peptides these polymers are of special interests with regards to achieve a controlled release of the active agent. Whang et al (2000) disclose an emulsion freeze-drying process starting with a PLA solution in methylene chloride for the fabrication of a biodegradable scaffold capable of incorporating and delivering bioactive macromelecules for bone regeneration. Schmidmaier et al (2000) disclose the use of a chloroform solution of PLA together with the osteoinductive factors IGF-I and TGF-1 in the coating implants.

WO02/070029 discloses a porous β-TCP matrix which is optionally admixed with PLGA microspheres encapsulated with OP-1 (osteogenic protein 1, a bone morphogenic protein) to form a heterogeneous material. In contrast to WO 03/043673 the β-TCP matrix in WO02/070029 exhibits single separate voids instead of interconnected pores. The pores of this matrix are not capable to be equipped with a homogeneous coating of the polymer and/or active agent component. The microspheres are produced by Alkmeres, Inc and exhibit a 20 to 500 µm diameter permitting microaggregation of the encapsulated active agent. For the production of such microspheres methylene chloride solution of the polymeric component together with the protein are sprayed and frozen in a deeply cold ethanol. (Herbert, P. et al. 1998 and see e.g. US Patent Application No 6,726,860), both steps in combination with two different organic solvents imparting chemical and mechanical stress to the protein.

For the manufacturing of larger specimen made from PLGA different techniques are used. Most of these techniques are adapted from standard polymer processing like hot melt extrusion or melt pressing. (Breitenbach, J. 2002) These techniques allow cost effective preparation and yield, in some cases to specimens with excellent mechanical properties (Törmälä, P. et al. 1992) for load bearing implants like screws or plates for bone fixation for example. Nevertheless these techniques are not designed to incorporate sensitive molecules like proteins or peptides. During these harsh manufacturing conditions the molecules would be stressed which would leadto an intolerable amount of protein degradation.

Therefore there is still need for a technique to incorporate process sensitive molecules into scaffolds or solid three dimensional specimens without process induced protein or peptide degradation and a need for materials obtained with such techniques.

Accordingly, an object underlying the present invention is the provision of a material, preferably a solid three dimensional material, suitable for implantation into a subject in the need of bone augmentation, which material allows a retarded release of an attached active agent and preferably further optimized local activity of an active agent.

Another object underlying the present invention is the provision of a material, preferably a solid three dimensional material, suitable for implantation into a subject in the need of bone augmentation allowing retarded release of an attached active agent and avoiding the problems associated with a local pH decrease induced by polymer degradation.

Another object underlying the present invention is the provision of a material, preferably a solid three dimensional material, suitable for implantation into a subject in the need of bone augmentation allowing retarded release of an attached active agent and avoiding toxic side effects and / or inflammatory responses.

Another object underlying the present invention is the provision of a material, preferably a solid three dimensional material, suitable for implantation into a subject in the need of bone augmentation allowing retarded release of an attached active agent and allowing lower doses of the active agent compared to conventional devices.

Another object underlying the present invention is the provision of a material, preferably a solid three dimensional material, suitable for implantation into a subject in the need of bone, allowing retarded release of an attached active agent and having the manifestation of a load bearing three-dimensional implant with mechanical properties preferably similar to bone.

### Summary of the Invention

Surprisingly, the present inventors were able to provide a material solving these objects and corresponding methods for the production of said material.

Accordingly, the present invention provides a method for the production of a material comprising the steps of:
(a) providing a solution comprising an active agent, and a polymer, wherein said active agent and said polymer are kept dissolved for a time sufficient to allow homogenous coating of a carrier, preferably a ceramic carrier, when said carrier is contacted with said solution;
(b) contacting the solution of step (a) with a carrier, preferably a ceramic carrier, more preferably a ceramic carrier containing calcium phosphate
(c) allowing homogenous coating of the surface of said carrier with said dissolved active agent and polymer;
(d) drying the coated carrier obtained in step (c); and
(e) thermally treating said coated carrier obtained in step (d).

This first method ("method A") is suitable for an active agent soluble in and compatible with an organic polymer solutions.

Additionally, a method for the production of a material comprising the steps of:
(a) providing an aqueous solution comprising an active agent and a buffer, which buffer keeps said active agent dissolved for a time sufficient to allow homogenous coating of a carrier, preferably a ceramic carrier when said carrier is contacted with said solution;
(b) contacting the solution of step (a) with a carrier, preferably a ceramic carrier, preferably a ceramic carrier containing calcium phosphate;
(c) allowing homogenous coating of the surface of said carrier with said dissolved active agent;
(d) drying the coated carrier obtained in step (c);
(e) providing a further solution comprising dissolved polymer, which polymer stays dissolved for a time sufficient to allow homogenous coating of the carrier obtained in step (d) when said carrier is contacted with said solution, wherein the active agent coated onto said carrier is not soluble in said solution;
(f) drying the polymer coated carrier obtained in step (e); and
(g) thermally treating said coated carrier obtained in step (f) is provided.

This second method ("method B") is suitable also for an active agent insoluble in or incompatible with an organic polymer solution.

The present invention further relates to a material produced by one of said methods, which material has osteoinductive and osteoconductive properties in vivo, comprises a carrier, wherein an active agent, preferably an osteoinductive protein, is coated onto the carrier together with a polymer, preferably a biodegradable polymer.

### Brief description of the Figures

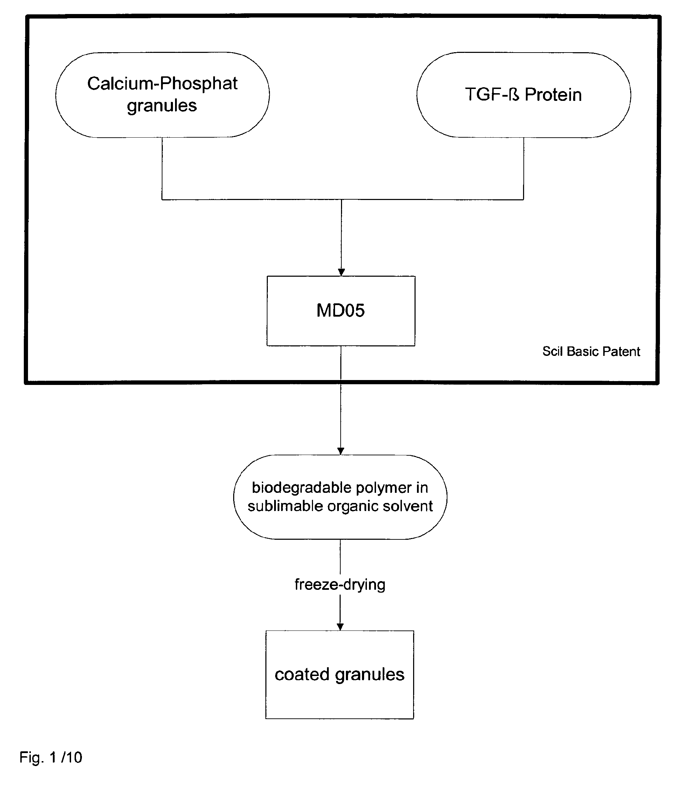

### Figure 1: Composite material for use as protein carrier

The production process in a preferred embodiment of the present invention (i.e. for Calcium Phosphate based porous granules) in comparison to the production process set forth in WO 03/043673 is schematically shown.

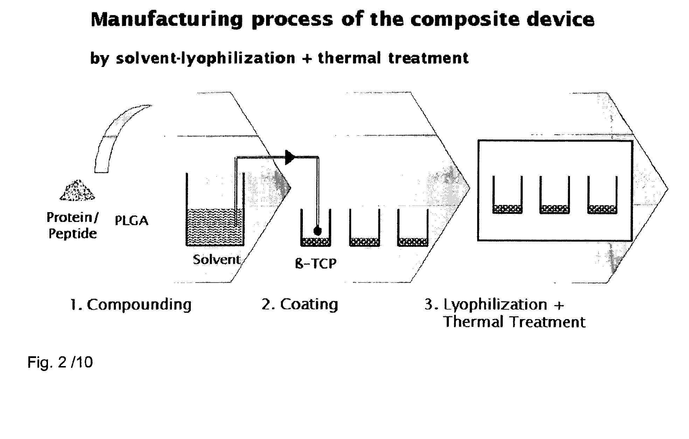

### Figure 2: Manufacturing process composite material

The process flow chart shows the three steps of the manufacturing process:
Step A: dissolution of the polymer and eventually the active agent in a suitable organic solvent.
Step B: coating the ceramic material with polymer and/ore active agent by soaking and subsequent drying.
Step C: After removal of the solvent the thermal treatment leads to a defined polymeric shell

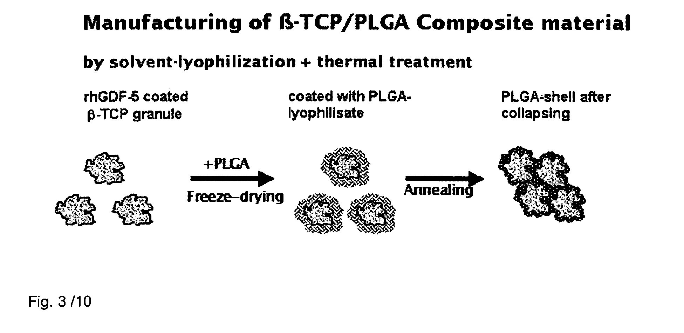

### Figure 3: Manufacturing of composite material

Step A: shows the protein loaded ceramic granule schematically in a cross sectional view
Step B: shows the protein loaded ceramic granule, with freeze dried polymer coat schematically in a cross sectional view
Step C: shows the protein loaded ceramic granule, with the defined polymeric shell achieved by thermal treatment schematically in a cross sectional view

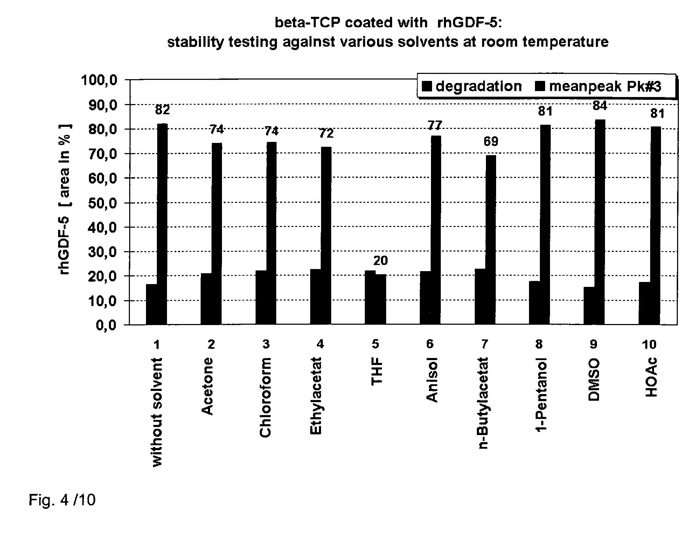

### Figure 4: Stability of rhGDF-5 on β-TCP in contact with various organic solvents

Fig. 4 shows the results of solvent screening: The stability of rhGDF-5 on β-TCP in contact with various organic solvents after drying at 25 °C he selected solvents are DMSO, glacial acetic acid and anisole.

The graph shows the content of modified species after contacting rhGDF-5 bound onto beta-TCP with various organic solvents at room temperature for 30 minutes. The solvents tested are acetone, chloroform, ethyl acetate, tetrahydrofurane, anisole, n-butylacetate, 1-pentanol, dimethylsulfoxide, glacial acetic acid

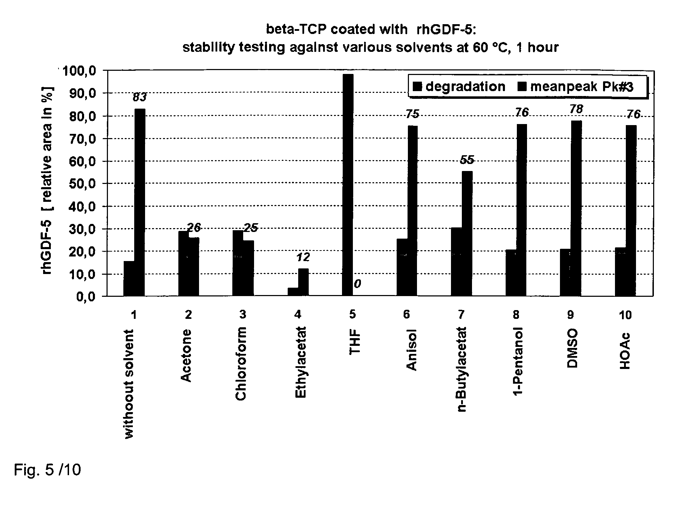

### Figure 5: Stability of rhGDF-5 on β-TCP in contact with various organic solvents and annealing.

The graph shows the content of modified species after contacting rhGDF-5 bound onto beta-TCP with various organic solvents at room temperature for 30 minutes. After the subsequent dying step the remaining protein coated granules were incubated at 60 °C to simulate the conditions at thermal treatment process. The solvents tested are acetone, chloroform, ethyl acetate, tetrahydrofurane, anisole, n-butylacetate, 1-pentanol, dimethylsulfoxide (DMSO), glacial acetic acid

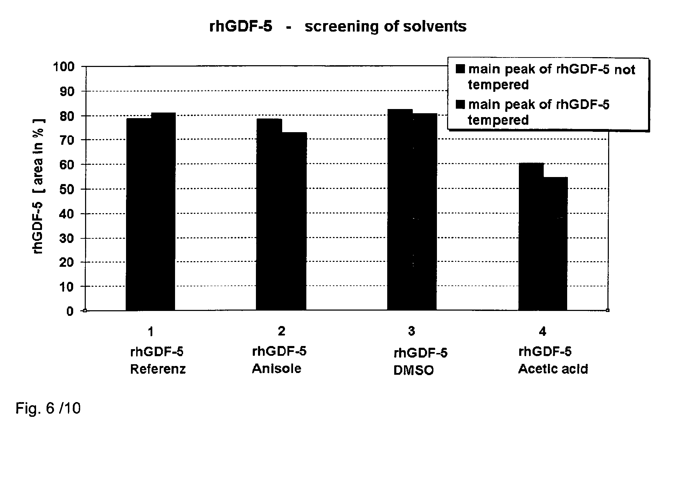

### Figure 6: Stability of pure rhGDF-5 in contact with various organic solvents and annealing

The graph shows the relative content of unmodified species after contacting pure rhGDF-5 with various organic solvents at room temperature for 30 minutes. After the subsequent dying step the remaining pure protein was incubated at 60 °C to simulate the conditions at thermal treatment process. The solvents tested are anisole, dimethylsulfoxide (DMSO), and glacial acetic acid.

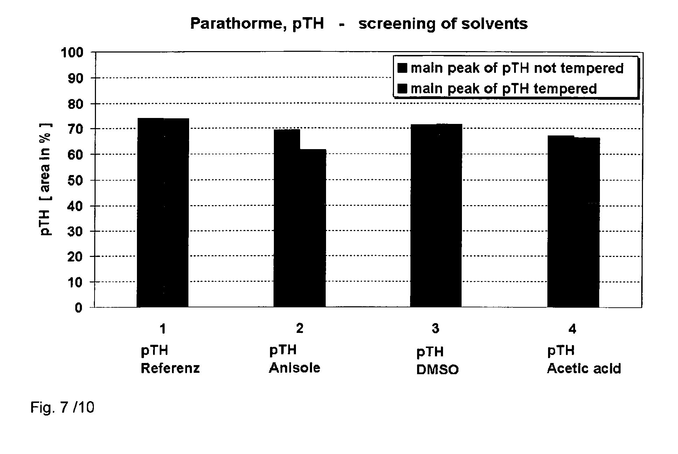

### Figure 7: Stability of pure parathormone in contact with various organic solvents and annealing

The graph shows the relative content of unmodified species after contacting pure parathormone PTH 1-34 with various organic solvents at room temperature for 30 minutes. After the subsequent dying step the remaining pure parathormone PTH 1-34 was incubated at 60 °C to simulate the conditions at thermal treatment process. The solvents tested are anisole, dimethylsulfoxide (DMSO), glacial acetic acid.

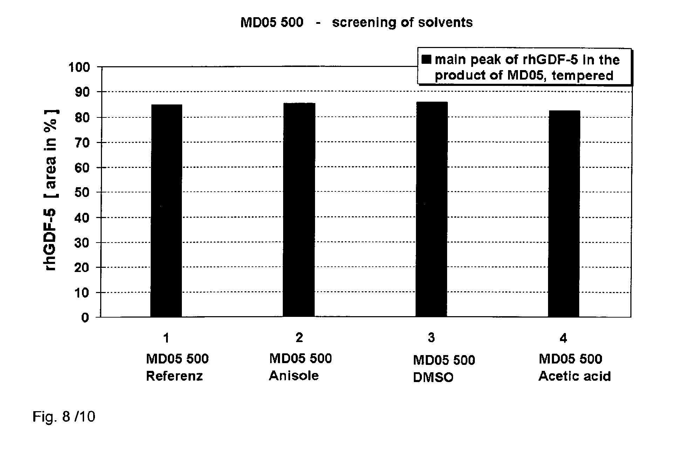

### Figure 8: Stability of rhGDF-5 on β-TCP in contact with various organic solvents and annealing after optimized conditions.

The graph shows the relative content of unmodified species after contacting the rhGDF-5 bound onto beta-TCP with various organic solvents at room temperature for 30 minutes. After the subsequent freeze drying step the remaining protein coated granules were incubated at 60 °C at high vacuum (≤ 0.1 mbar) to simulate the conditions at thermal treatment process. The solvents tested are anisole, dimethylsulfoxide (DMSO), and glacial acetic acid.

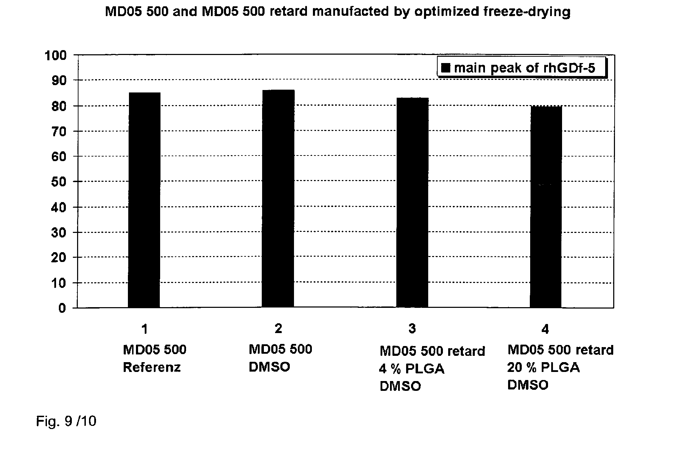

### Figure 9: Stability of PLGA coated rhGDF-5 on β-TCP in contact with various organic solvents and annealing after optimized conditions.

The graph shows the relative content of unmodified species after contacting the rhGDF-5 bound onto beta-TCP with a solution of PLGA in various organic solvents at room temperature for 30 minutes. After the subsequent freeze dying step the remaining protein coated granules were incubated at 60°C at high vacuum (≤ 0.1 mbar) for thermal treatment process to achieve the defined polymeric shell. The solvents tested are anisole, dimethylsulfoxide (DMSO), and glacial acetic acid.

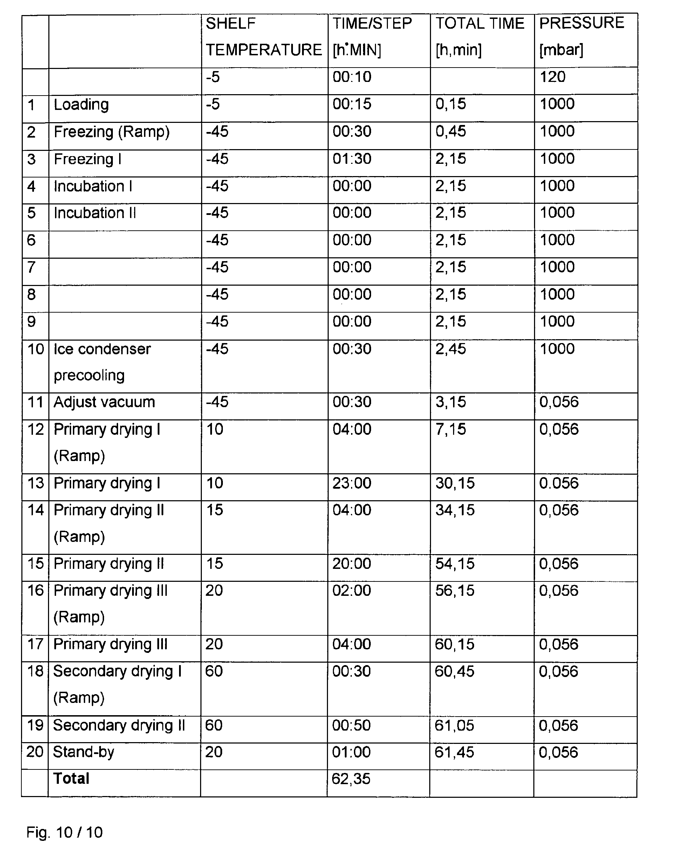

### Figure 10: Timetable showing lyophilisation details

This table shows the details of the lyophilization program for the manufacturing of protein or peptide PLGA loaded composite PLGA/β-TCP granules to achieve a minimum solvent induced protein- or peptide degradation.

### Detailed description of the preferred embodiments

### Detailed description of the preferred embodiments

The term "material" as used in accordance to the present invention refers to an entity, which comprises at least three components as set forth below. In one embodiment the material is a drug delivery system with porous scaffold. This material is preferably suitable for surgical defect filling and tissue regeneration. In another embodiment the material is a drug delivery system for the controlled release of active substances after implantation.

One of the components of said material is an inorganic matrix, the so-called "carrier". Preferably, said inorganic matrix consists of ceramics. More preferably, said carrier is a calcium phosphate. Most preferably said inorganic matrix is a calcium phosphate, which is beta tricalcium phosphate, alpha tricalcium phosphate, apatite or a calcium phosphate containing cement. Alternatively, said carrier is selected from the group consisting of calcium carbonate, magnesium carbonate, magnesium oxide or magnesium hydroxide.

Said ceramics may have a particularly high surface due to the small particles size or the presence of macro- and micropores. In a preferred embodiment, said macropores have a diameter of 100 to 400 µm. In another preferred embodiment, said micropores have a diameter of less than 10 µm. In still another preferred embodiment, the pores are interconnected to allow the influx of coating substances in the material preparation as well as in-growth of bone and tissue cells in the application in vivo.

The term "carrier" encompasses three-dimensional matrices, such as the above-mentioned ceramics and ceramic/polymer composites. The carrier, preferably, has an enlarged surface due to the small particles size or the formation of macro- and micropores during the manufacturing process.

The carrier comprised by the material of the invention may be brought into a suitable form for administration of the material *in vivo,* such as solid composite materials in form of blocks, cubes, discs or granules. In addition, the composite carrier may be coated onto a metallic surface.

In a preferred embodiment, the carrier containing calcium phosphate is in a granular form, more preferably in the form of free flowing granules. Granular products as moldable systems are well established for surgical defect filling especially in orthopedic indications (Draenert et al.). Therefore it is important to meet this preferred application form. In an alternatively preferred embodiment this granular form is used as a starting material for forming a solid three dimensional scaffold, preferably with the manifestation of a load bearing three-dimensional implant with mechanical properties preferably similar to bone. This solid three dimensional scaffold is preferably formed by annealing the granules.

The term "calcium phosphate" encompasses compositions comprising calcium ions (Ca²⁺), phosphate ions (PO₃³⁻), optionally, further ions like hydroxyl ions (OH⁻), carbonate (CO₃²⁻) or magnesium (Mg²⁺) or other ions which are suitable for the carrier of the present invention. The calcium phosphates as used in accordance with the present invention are crystals having a three dimensional structure suitable for the material of the present invention as set forth above. Said calcium phosphates are particularly well suited as carriers for the material of the present invention. Their in vivo properties have been described in Hotz, 1994, Gao, 1996, and in WO98/21972. A list of preferred and well-known calcium phosphates is given above.

The second component of the material of the present invention is a polymer. Preferably said polymer is a "biocompatible", a "biodegradable" or a "bioresorbable" polymer.

The term "biocompatible" means the ability of a material to perform with an appropriate host response in a specific application (Wintermantel E. et al., 2002). "Biodegradable polymers" specifies polymers, which break down due to macromolecular degradation with dispersion in vivo but for which no proof exists for the elimination from the body. The term "bioresorbable polymer" specifies polymeric materials, which underwent degradation and further resorption in vivo; i.e. polymers, which are eliminated through natural pathways either because of simple filtration of degradation by-products or after their metabolization. Bioresorption is thus a concept which reflects total elimination of the initial foreign material.

In a preferred embodiment of the material or the method of the invention said bioresorbable polymer is a polymer that undergoes a chain cleavage due to macromolecular degradation in an aqueous environment.

Alternatively, said polymer is selected from the group consisting of polyethylene (PE), polypropylene (PP), polyethylenerephthalate (PET), polyglactine, polyamide (PA), polymethylmethacrylate (PMMA), polyhydroxymethylmethacrylate (PHEMA), polyvinylchloride (PVC), polyvinylalcohole (PVA), polytetrafluorethylene (PTFE), polyetheretherketone (PEEK), polysulfon (PSU), polyvinylpyrolidone, polyurethane or polysiloxane. These polymers are at least biocompatible.

More preferably, said polymer is selected from the group consisting of poly(α-hydroxy acids), poly (ortho esters), poly(anhydrides), poly(aminoacids), polyglycolid (PGA), polylactid (PLLA), poly(D,L-lactide) (PDLLA), poly(D,L-lactide co-glycolide) PLGA), poly(3-hydroxybutyricacid) (P(3-HB)), poly(3-hydroxy valeric acid) (P(3-HV)), poly(p-dioxanone) (PDS), poly(ε-caprolactone) (PCL), polyanhydride (PA). These polymers are biocompatible and bioresorbable.

The third component of the material of the present invention is an "active agent". The term "active agent" comprises a polypeptide or a small molecule drug which is immobilized on and/or in the carrier. Preferably, said polypeptide or drug is homogeneously distributed on the calcium phosphate containing carrier and / or within the polymer.

The term "osteoconductive" refers to substrates that provide a favourable scaffolding for vascular ingress, cellular infiltration and attachment, cartilage formation, and calcified tissue deposition. Osteoconductive materials may support osseous generation via the scaffolding effect (Kenley, R.A., 1993).

The term "osteoinductive" refers to the capability of the transformation of mesenchymal stem cells into osteoblasts and chondrocytes. A prerequisite for osteoinduction is a signal which is distributed by the material into the surrounding tissues where the aforementioned osteoblast precursors become activated. Osteoinduction as used herein encompasses the differentiation of mesenchymal cells into the bone precursor cells, the osteblasts. Moreover, osteoinduction also comprises the differentiation of said osteoblasts into osteocytes, the mature cells of the bone. Moreover, also encompassed by osteoinduction is the differentiation of mesenchymal cells into chondrocytes. In particular in the long bones, the chondroblasts and the chondrocytes residing in the perichondrium of the bone can also differentiate into osteocytes. Thus, osteoinduction requires differentiation of undifferentiated or less-differentiated cells into osteocytes which are capable of forming the bone. Thus, a prerequisite for osteoinduction is a signal which is distributed by the material into the surrounding tissues where the aforementioned osteocyte precursors usually reside. As has been described above, the osteoinductive proteins or peptides used in accordance with the present invention are sustained released from the material after implantation and are distributed efficiently in the surrounding tissues. Moreover, the proteins and peptides encompassed by the present invention have osteoinductive properties in vivo. For example, it is well known in the art that the Transforming Growth Factor-β (TGF-β) superfamily encompasses members which have osteoinductive properties. Individual members of said TGF-β superfamily which have particular well osteoinductive properties are listed infra. In conclusion, the osteoinductive proteins or peptides of the material of the present invention after having been released from the carrier serving as a osteoinductive signal for the osteocyte precursors of the tissue surrounding the side of implantation of the material.

The term "osteogenic" describes the synthesis of new bone by osteoblasts. In accordance with the present invention, preexisting bone in the surrounding of the side of implantation of the material grows into the material using the structure of the material as a matrix onto which the osteocytes can adhere.

The term "osteoinductive polypeptide" refers to polypeptides, such as the members of the Transforming Growth Factor-β (TGF-β) superfamily, which have osteoinductive properties.

In a further preferred embodiment of the material or the method of the invention said osteoinductive protein is a member of the TGF-β family.

The TGF-β family of growth and differentiation factors has been shown to be involved in numerous biological processes comprising bone formation. All members of said family are secreted polypeptides comprising a characteristic domain structure. On the very N-terminus, the TGF-β family members comprise a signal peptide or secretion leader. This sequence is followed at the C-terminus by the prodomain and by the sequence of the mature polypeptide. The sequence of the mature polypeptide comprises seven conserved cysteins, six of which are required for the formation of intramolecular disulfide bonds whereas one is required for dimerization of two polypeptides. The biologically active TGF-β family member is a dimer, preferably composed of two mature polypeptides. The TGF-β family members are usually secreted as proteins comprising in addition to the mature sequence the prodomain. The prodomains are extracellularly cleaved off and are not part of the signalling molecule. It has been reported, however, that the prodomain(s) may be required for extracellular stabilization of the mature polypeptides.

In the context of the present invention, the term "TGF-β family member" or the proteins of said family referred to below encompass all biologically active variants of the said proteins or members and all variants as well as their inactive precursors. Thus, proteins comprising merely the mature sequence as well as proteins comprising the mature protein and the prodomain or the mature protein, the prodomain and the leader sequence are within the scope of the invention as well as biologically active fragments thereof. Whether a fragment of a TGF-β member has the biological activity can be easily determined by biological assays described, e.g. in: Katagiri T, Yamaguchi A, Ikeda T, Yoshiki S, Wozney JM, Rosen V, Wang EA, Tanka H, Omura S, Suda T, (1990): The non-osteogenic mouse pluripotent cell line, C3H10T1/2, is induced to differentiate into osteoblastic cells by recombinant human bone morphogenetic protein-2. Biochem. Biophys. Res. Commun. 172: 295-299 or Nishitoh H, Ichijo H, Kimura M, Matsumoto T, Makishima F, Yamaguchi A, Yamashita H, Enomoto S, Miyazono K (1996): Identification of type ( and type (( serine/ threonine kinase receptors for growth/ differentiation factor-5. J. Biol. Chem. 271: 21345-21352.

Preferably, the biological activity according to the invention can be determined by in vivo models as described in the accompanied Examples. Furthermore, encompassed by the present invention are variants of the TGF-β members which have an amino acid sequences being at least 75%, at least 80%, at least 90%, at least 95%, at least 96%, at least 97%, at least 98% or at least 99% identical to the amino acid sequences of the members of the TGF-β family.

An overview of the members of the TGF-β superfamily is given in: Wozney JM, Rosen V (1998): Bone morphogenetic protein and bone morphogenetic protein gene family in bone formation and repair. Clin Orthop 346: 26-37. The amino acid sequences of the members of the TGF-β family can be obtained from the well known databases such as Swiss-Prot via the internet (http://www.expasy.ch/sprot/sprot-top.html) Amino acid sequences for BMP2, BMP7 and GDF-5, members of the TGF-( family with a particularly high osteoinductive potential, are also shown in SEQ ID No: 1 to 3, respectively. Amino acid sequences for BMP2, BMP7 and GDF-5, members of the TGF-β family with a particularly high osteogenic potential, are also shown in SEQ ID No:1 to 3, respectively.

More preferably, said member of the TGF-β family is a member of the BMP subfamily.
The members of the Bone Morphogenetic Protein (BMP) subfamily have been shown to be involved, inter alia, in the induction and re-modeling of bone tissue. BMPs were originally isolated from bone matrix. These proteins are characterized by their ability to induce new bone formation at ectopic sites. Various in vivo studies demonstrated the promotion of osteogenesis and chondrogenesis of precursor cells by BMPs and raise the possibility that each BMP molecule has distinct role during the skeletal development. More details about the molecular and biological properties of the BMPs are described in:
Wozney JM, Rosen V (1998): Bone morphogenetic protein and bone morphogenetic protein gene family in bone formation and repair. Clin Orthop 346: 26-27, Schmitt J, Hwang K, Winn, SR, Hollinger J (1999): Bone morphogenetic proteins: an update on basic biology and clinical relevance. J Orthop Res 17: 269-278 and Lind M (1996): Growth factors: possible new clinical tools. A review. Acta Orthop Scand 67: 407-17.

The osteoinductive polypeptide of the present invention is preferably selected from the group consisting of BMP-1, BMP2, BMP-3, BMP-4, BMP-5, BMP-6, BMP7, BMP-8, BMP-9, BMP-10, BMP-11, BMP-12, BMP-13, BMP-14, BMP-15 and BMP-16. Most preferably, said member of the BMP family is BMP2 or BMP7.

The amino acid sequence for the preproform of BMP2 is deposited under Swiss-Prot Accession number P12643 and is shown below. Amino acids 1 to 23 correspond to the signal sequence, amino acids 24 to 282 correspond to the propeptide and amino acids 283 to 396 correspond to the mature protein. The amino acid sequence for the preproform of BMP7 is deposited under Swiss-Prot Accession number P18075 or shown in SEQ ID No: 2. Amino acids 1 to 29 correspond to the leader sequence, amino acids 30 to 292 correspond to the proform and amino acids 293 to 431 correspond to the mature protein. Preferably, BMP-2 or BMP7 refers to the preproform, to the proform or to the mature BMP-2 or BMP-7 peptide, respectively. Moreover also encompassed are fragments of said proteins having essentially the same biological activity, preferably osteoinductive properties. More sequence information for BMP2 and BMP7 is provided below.

Alternatively, the osteoinductive polypeptide of the present invention is selected from another TGF- β family, i.e. the GDF family.

Growth and Differentiation Factor (GDF) have been also shown to be involved, inter alia, in the induction and re-modeling of bone tissue. Growth Differentiation Factor 5 (GDF-5), also known as cartilage-derived morphogenetic protein 1 (CDMP-1) is a member of subgroup of the BMP family, which also includes other related proteins, preferably, GDF-6 and GDF-7. The mature form of the protein is a 27 kDa homodimer. Various in vivo and in vitro studies demonstrate the role of GDP-5 during the formation of different morphological features in the mammalian skeleton. Mutations of GDF-5 are responsible for skeletal abnormalities including decrease of the length of long bones of limbs, abnormal joint development in the limb and sternum (Storm & Kingsley (1999), Development Biology, 209, 11-27). The amino acid sequence between mouse and human is highly conserved.

Preferably, the osteoinductive polypeptide of the present invention is selected from the group consisting of GDF-1, GDF-2, GDF-3, GDF-4, GDF-5, GDF-6, GDF-7, GDF-8, GDF-9, GDF-10 and GDF-11. Most preferably, said member of the GDF subfamily is GDF-5.
The amino acid sequence for the preproform of GDF-5 is deposited under Swiss-Prot Accession number P 43026 or shown in SEQ ID No: 3. Amino acids 1 to 27 correspond to the leader sequence, amino acids 28 to 381 correspond to the proform and amino acids 382 to 501 correspond to the mature protein. Preferably, GDF-5 refers to the preproform, to the proform or to the mature GDF-5 peptide. Moreover also encompassed are fragments of GDF-5 having essentially the same biological activity, prefrably osteoinductive properties.

Most preferably, said fragment comprises amino acids 383 to 501 of the sequence shown in SEQ ID No: 3.

The following tables show amino acid sequences for BMP-2, BMP-7 and GDF-5:

Human BMP-2 (Swiss-Prot Prim. Accession Number P12643); SEQ ID No. 1:

Key
From To
Length

SIGNAL
1 23
23

PROPEP
24 282
259

hBMP2
283 396
114

10 20 30 40 50 60
MVAGTRCLLA LLLPQVLLGG AAGLVPELGR RKFAAASSGR PSSQPSDEVL SEFELRLLSM

70 80 90 100 110 120
FGLKQRPTPS RDAVVPPYML DLYRRHSGQP GSPAPDHRLE RAASRANTVR SFHHEESLEE

130 140 150 160 170 180
LPETSGKTTR RFFFNLSSIP TEEFITSAEL QVFREQMQDA LGNNSSFHHR INIYEIIKPA

190 200 210 220 230 240
TANSKFPVTR LLDTRLVNQN ASRWESFDVT PAVMRWTAQG HANHGFVVEV AHLEEKQGVS

250 260 270 280 290 300
KRHVRISRSL HQDEHSWSQI RPLLVTFGHD GKGHPLHKRE KRQAKHKQRK RLKSSCKRHP

310 320 330 340 350 360
LYVDFSDVGW NDWIVAPPGY HAFYCHGECP FPLADHLNST NHAIVQTLVN SVNSKIPKAC

370 380 390
CVPTELSAIS MLYLDENEKV VLKNYQDMVV EGCGCR

References

[1]

SEQUENCE FROM NUCLEIC ACID.
MEDLINE=89072730; PubMed=3201241;
Wozney J.M., Rosen V., Celeste A.J., Mitsock L.M., Whitters M.J., Kriz
R.W., Hewick R.M., Wang E.A.;
"Novel regulators of bone formation: molecular clones and activities.";
Science 242:1528-1534(1988).

[2]

X-RAY CRYSTALLOGRAPHY (2.7 ANGSTROMS) OF 292-396.
MEDLINE=99175323; PubMed=10074410;
Scheufler C., Sebald W., Huelsmeyer M.;
"Crystal structure of human bone morphogenetic protein-2 at 2.7 A
resolution.";
J. Mol. Biol. 287:103-115(1999).

Human BMP-7(Swiss-Prot Prim. Accession. Number: P18075); SEQ ID No. 2:

Key
From To
Length

SIGNAL
1 29
29

PROPEP
30 292
263

hBMP-7
293 431
139

10 20 30 40 50 60
| | | | | |
MHVRSLRAAA PHSFVALWAP LFLLRSALAD FSLDNEVHSS FIHRRLRSQE RREMQREILS

70 80 90 100 110 120
| | | | | |

ILGLPHRPRP HLQGKHNSAP MFMLDLYNAM AVEEGGGPGG QGFSYPYKAV FSTQGPPLAS

130 140 150 160 170 180
| | | | | |
LQDSHFLTDA DMVMSFVNLV EHDKEFFHPR YHHREFRFDL SKIPEGEAVT AAEFRIYKDY

190 200 210 220 230 240
| | | | | |
IRERFDNETF RISVYQVLQE HLGRESDLFL LDSRTLWASE EGWLVFDITA TSNHWVVNPR

250 260 270 280 290 300
| | | | | |
HNLGLQLSVE TLDGQSINPK LAGLIGRHGP QNKQPFMVAF FKATEVHFRS IRSTGSKQRS

310 320 330 340 350 360
| | | | | |
QNRSKTPKNQ EALRMANVAE NSSSDQRQAC KKHELYVSFR DLGWQDWIIA PEGYAAYYCE

370 380 390 400 410 420
| | | | | |
GECAFPLNSY MNATNHAIVQ TLVHFINPET VPKPCCAPTQ LNAISVLYFD DSSNVILKKY

430
|
RNMVVRACGC H

References

[1]

SEQUENCE FROM NUCLEIC ACID, AND PARTIAL SEQUENCE.
TISSUE=Placenta;
MEDLINE=90291971; PubMed=2357959;
Oezkaynak E., Rueger D.C., Drier E.A., Corbett C., Ridge R.J., Sampath
T.K., Oppermann H.;
"OP-1 cDNA encodes an osteogenic protein in the TGF-beta family.";
EMBO J. 9:2085-2093(1990).

[2]

SEQUENCE FROM NUCLEIC ACID.
MEDLINE=91088608; PubMed=2263636;
Celeste A.J., Iannazzi J.A., Taylor R.C., Hewick R.M., Rosen V., Wang E.A.,
Wozney J.M.;
"Identification of transforming growth factor beta family members present
in bone-inductive protein purified from bovine bone.";
Proc. Natl. Acad. Sci. U.S.A. 87:9843-9847(1990).

[3]

X-RAY CRYSTALLOGRAPHY (2.8 ANGSTROMS) OF 293-431.
MEDLINE=96149402; PubMed=8570652;
Griffith D.L., Keck P.C., Sampath T.K., Rueger D.C., Carlson W.D.;
"Three-dimensional structure of recombinant human osteogenic protein 1:
structural paradigm for the transforming growth factor beta superfamily.";
Proc. Natl. Acad. Sci. U.S.A. 93:878-883(1996).

Human GDF-5 (Swiss-Prot Prim. Accession Number: P 43026); SEQ ID No. 3:

Key
From To
Length

SIGNAL
1 27
27

PROPEP
28 381
354

hGDF-5
382 501
120

10 20 30 40 50 60
| | | | | |
MRLPKLLTFL LWYLAWLDLE FICTVLGAPD LGQRPQGSRP GLAKAEAKER PPLARNVFRP

70 80 90 100 110 120
| | | | | |
GGHSYGGGAT NANARAKGGT GQTGGLTQPK KDEPKKLPPR PGGPEPKPGH PPQTRQATAR

130 140 150 160 170 180
| | | | | |
TVTPKGQLPG GKAPPKAGSV PSSFLLKKAR EPGPPREPKE PFRPPPITPH EYMLSLYRTL

190 200 210 220 230 240
| | | | | |
SDADRKGGNS SVKLEAGLAN TITSFIDKGQ DDRGPVVRKQ RYVFDISALE KDGLLGAELR

250 260 270 280 290 300
| | | | | |
ILRKKPSDTA KPAVPRSRRA AQLKLSSCPS GRQPAALLDV RSVPGLDGSG WEVFDIWKLF

310 320 330 340 350 360
| | | | | |
RNFKNSAQLC LELEAWERGR TVDLRGLGFD RAARQVHEKA LFLVFGRTKK RDLFFNEIKA

370 380 390 400 410 420
| | | | | |
RSGQDDKTVY EYLFSQRRKR RAPLATRQGK RPSKNLKARC SRKALHVNFK DMGWDDWIIA

430 440 450 460 470 480
| | | | | |
PLEYEAFHCE GLCEFPLRSH LEPTNHAVIQ TLMNSMDPES TPPTCCVPTR LSPISILFID

490 500
| |
SANNVVYKQY EDMVVESCGC R

References

[1]

SEQUENCE FROM NUCLEIC ACID.
TISSUE=Placenta;
MEDLINE=95071375; PubMed=7980526;
Hoetten G., Neidhardt H., Jacobowsky B., Pohl J.;
"Cloning and expression of recombinant human growth/differentiation factor
5.";
Biochem. Biophys. Res. Commun. 204:646-652(1994).

[2]

SEQUENCE FROM NUCLEIC ACID.
TISSUE=Articular cartilage;
MEDLINE=95050604; PubMed=7961761;
Chang S., Hoang B., Thomas J.T., Vukicevic S., Luyten F.P., Ryba N.J.P.,
Kozak C.A., Reddi A.H., Moos M.;
"Cartilage-derived morphogenetic proteins. New members of the transforming
growth factor-beta superfamily predominantly expressed in long bones during
human embryonic development.";
J. Biol. Chem. 269:28227-28234(1994).

Also encompassed by the present invention are embodiments, wherein said active agent is selected from hormones, cytokines, growth factors, antibiotics and other natural and/or synthesized drug substances like steroids, prostaglandines etc.

Preferably, said active agent is parathyroid hormone (PTH) and/or PTH 1-34 peptide.

The material of the present invention may optionally, comprise additional excipients. These excipients serve the stabilization of the protein or peptide, e.g., saccharides, amino acids, polyols, detergents or maintenance of the pH, e.g., buffer substances.

In a preferred embodiment the material of the present invention is free of toxic substances. Preferably such toxic substances are already avoided in the production process, as their production requires additional expenditure due to required removal steps during the production process and necessary expensive means for highly sensitive chemical analysis.

The term "toxic substances", in particular, encompasses those toxic organic solvents and additives which are used by the methods described in the art, which are classified by the ICH as class 2 solvents (ICH Topic Q 3 C Impurities: Residual Solvents) e.g. methylene chloride. Said substances may cause systemic or local toxic effects, inflammation and / or other reactions after implantation of materials containing said substances. Said prior art materials are therapeutically less acceptable due to said undesirable side effects, which cannot be avoided by the conventionally coating methods described in the art. Moreover, the international guidance for the development of therapeutic proteins require that in the manufacturing process harmful and toxic substances should be avoided (for details see: International Conference on Harmonization (ICH), Topic Q3C; www.emea.eu.int/). However, the material of the present invention or a material which is obtainable by the method of the present invention is, advantageously, free of said class 1 classified toxic substances. Moreover the present invention contains only solvents classified as class 3 by the ICH Topic Q 3C and, therefore, therapeutically well acceptable and fulfills the requirements of the regulatory authorities.

Moreover, in a further preferred embodiment of the material or the method of the invention said material is free of infectious material.

Besides toxic substances, infectious material comprised by the material may cause severe infections in a subject into which the material has been transplanted. Potentially infectious gelatin derived from bovine or procine bones is, however, used as a protecting protein in many state of the art methods (Lind, 1996).

Solutions sufficient for the two methods A and B of the present invention (used in step (a) of method A and step (e) of method B, respectively) to produce the material of the present invention have a melting point > -40 °C and a boiling point < 200 °C. They are inactive towards the active agent.

Preferable this solution contains a solvent selected from anisole, tetramethylurea, acetic acid, dimethylsulfoxide and tert-butanol (2-methyl-2-propanole trimethylcarbinole-butyl alcohol), acetone, 1-butanole, 2-butanole, butyl acetate, tert-butylmethyl ether, cumene, ethanole, ethyl acetate, dieethylether, ethylformate, formic acid, isobutyl acetate, isopropylacetate, methyl acetate, 3-methyl-1-butanol, methylethyl ketone, methylisobutylketone, 2-methyl-1-propanol, pentane, 1-pentanol, 2-propanol, propylacetate, and tetrahydrofurane. Most preferred are acetic acid, dimethylsulfoxide and anisole.

The terms "homogeneously distributed" and "homogeneously coateded" mean that identical amounts of the active agent are present in each and every area of said composite carrier. This area preferably includes the pores of a porous matrix. Homogenous distribution is a prerequisite for efficient release and activity of the active agent into the tissue surrounding the site of implantation. Moreover, it is to be understood that the active agent is not aggregated and partially or entirely inactivated due to precipitation or micro-precipitation, rather attachment of biologically active, non-aggregated proteins is to be achieved by homogenous coating. Said homogenous distribution can be achieved by the two above methods of the present invention.

The homogenous coating of the carrier with said active agent and the simultaneous and / or additional homogeneous coating with the bioresorbable polymer do achieve an onion-like layer structure which acts in two manners as a protective film and as diffusion barrier to slow down the dissolution of the protein or peptide to achieve a sustained release. The described methods A and B allow the homogenous distribution and immobilization of the osteoinductive active agent into and / or on the carrier and the sustained release of the active agent due to the polymeric component.

The efficacy of the coating process is, furthermore, supported by the carrier due to capillary forces resulting from the presence of numerous, preferably interconnected macro- and micropores which due to their size are capable of soaking the solutions into the pores.

Moreover, in contrast to other methods described in the art, e.g., in WO98/21972, the active agent is - according to the methods A and B of the present invention - applied by attachment to the carriers from the soluble state to achieve a homogeneous coating. The findings underlying the present invention demonstrate that the aggregation of the proteins can be avoided in a tri-component-system by the use of suitable solvents and / or additives as described herein. An important precondition is the knowledge of the solubility of the osteoinductive active agent dependent on the nature of the solvent, i.e. aqueous and / or organic solvent, pH value, ionic strength and surfaces present.

The term "aqueous solution" specifies any solution comprising water. The slowing down of the pH increase caused by the contact of the coating solution with the calcium phosphates in the carrier reacting in an alkaline manner, in particular, plays an important role during the coating, preferably in method B.

The methods A and B of the present invention, distribute the active agent homogeneously across the inner surface of the carrier material and allow binding to the surface before a precipitation of the said protein takes place.

In method B this precipitation is pH-induced. It could be demonstrated that in this case the pH increase taking place during the coating of calcium phosphates is decelerated sufficiently by the use of a weak acid, such as acetic acid. Furthermore, the addition of organic compounds such as ethanol or sucrose proves to be additionally advantageous here. Furthermore, a low ionic strength is an important precondition for successful coating of the protein or peptide onto the calcium phosphate. Moreover, our tests show that the volume of the coating solutions (solution containing active agent and / or polymer), too, has a considerable effect on the quality of both coatings.

Finally, the methods A and B of the present invention allow the use of non toxic organic solvents (see below), such as dimethyl sulfoxide, anisol or glacial acid. These solvents are routinely used in the methods described in the art. Normally they damage the protein during contacting and/or especially during drying but such damage is surprisingly avoided by using the special drying technique of the present invention, because the active agent is adsorbed/or attached onto the inorganic solid carrier.

In a preferred embodiment of the method B of the invention said active agent coating buffer has a buffer concentration of preferably less than 100 mmol/l, more preferably less than 50 mmol/l and even more preferably less than 20 mmol/l to achieve a sufficient solubility of the active agent during the adsorbtion process and to avoid any modification of a ceramic carrier.

In another preferred embodiment of the method of the invention said buffer has a buffer concentration of 10 mmol/l to achieve a sufficient solubility of the active agent during the adsorbtion process and to avoid any modification of the monophasic calcium phosphate ceramic beta TCP. The pH of the solution shifts in a controlled manner during the coating and drying process from pH 3 to pH 7, more preferably from 3 to 6 and most preferably from 4 to 5,5. This pH shift causes a defined reduction of the solubility of the bone growth factor to result in a homogenous, defined attachment on the TCP.

In a preferred embodiment of the methods of the invention the solutions comprise non toxic organic solvents. The first aspect for method A is to find a common suitable organic solvent for both, the active agent and the polymer, without inducing modifications at the active agent. A second aspect is the ability of the solvent(s) in step (a) method A and / or step (e) of method B for the drying process, which is preferably a freeze-drying process. The preferred solvents are anisole, dimethylsulfoxide (DMSO) and glacial acetic acid. In a preferred embodiment these solvents in both method A and in method B are used in volumina to achieve a compleate soaking of the polymer solution and to avoid any remaining solution.

It follows from the above that preferably, said buffer contains a weak acid. The term "weak acid" refers to organic or inorganic compounds containing at least one ionogenically bound hydrogen atom. Weak acids are well known in the art and are described in standard text books, such as Römpp, "dictionary of chemistry". Preferably, said weak acids which have low dissociation degrees and are described by pK values between 3 and 7, preferably between 4 and 6. Most preferably, said weak acid is acetic acid or succinic acid.

In another preferred embodiment of method B of the invention said buffer containing solution further comprises at least one saccharides in an aqueous solution, more preferably in an aqueous solution without any further solvent apart from water.

In a further preferred embodiment of the method of the invention said buffer containing solution comprises an polyol and/or alcohol. Suitable alcohols or polyols are well known in the art and are described in standard text books, such as Römpp, dictionary of chemistry. More preferably, said alcohol is ethanol and said polyol is mannitol.

In a more preferred embodiment the concentration of the polyol and or alcohol is between 0 - and 10 % (w/v).

The term "saccharides" encompasses mono-, di- and polysaccharides. The structure and composition of mono-, di-, and polysaccharides are well known in the art and are described in standard text books, such as Römpp, "dictionary of chemistry".

More preferably, said saccharide is a disaccharide. Most preferably, said dissaccharide is sucrose or trehalose.

Further means and methods for controlling homogeneous distribution, quantification and characterization of the active agent are described in the accompanied examples.

Surprisingly, active agents, in particular surprisingly proteins, when adsorbed on the surface of ceramic carriers are much more resistant against degradation caused by organic solvents than proteins freely dissolved or suspended in organic solutions or integrated in biphasic emulsion systems. Thus, this aspect of the invention opens a new possibility to produce polymer based drug delivery systems for proteins without denaturation and / or modification of polypeptides in singular or multiphase organic systems, preferably for active agents incompatible with organic solvents.

Furthermore the type of ceramic carrier preferably used in present invention opens the possibility to quantitatively remove organic solvents (see below).

Suitable for one of the two methods of the present invention as active agents are all proteins, polypeptides and small molecule drugs. Especially such active agents with low or no affinity for inorganic carrier matrices can be immobilized in the polymer - calcium phosphate composite material. Preferably, the binding of said active agent to the carrier is reversible.

Thereby, dissolution of said active agent is allowed once the material has been brought into a suitable in vivo surrounding, such as a bone cavity. More preferably, said dissolution of the immobilized compounds is a sustained release allowing diffusion of the active agent into the tissue which surrounds the material. Thus, the material is suitable to serve as an in vivo source for e.g. osteoinductive proteins, peptides or small molecule drugs, which are slowly released and which can be thereby efficiently distributed into the surrounding tissues or have an effect in the immobilized form.

The term "drying" encompasses measures for removing liquids, such as excess buffer solution, or organic solvents which are still present after coating of the carrier with the osteoinductive protein or polymer solution. Preferably, drying is achieved by convection at under inert gas atmosphere, by vacuum- or freeze-drying. It is important for the composite ceramic of the present invention that after drying the ceramic matrix is substantially free of organic solvent to allow for a softening of the polymeric component in a thermal treatment step, such as steps (e) of method A and step (g) of method B. Substantially free of organic solvent means a content preferably below ≤ 1 % residual solvent, more preferably ≤ 0,05 %, even more preferably ≤ 0,025 % and most preferably ≤ 0,01 %.

The term "buffer" which assists in keeping the active agent dissolved in aqueous solutions for a time sufficient to allow "homogenous coating" refers to a component allowing the active agent to be effectively dissolved in the solution and / or homogeneously coated in a carrier system tending to cause pH induced precipitation. This buffer is preferably capable of avoiding and or balancing the increase of pH caused by contacting the solution with the calcium phosphate carrier so that the protein does not immediately precipitate, e.g., due to a pH increase. Said buffer can be determined by the person skilled in the art considering the solubility of the osteoinductive protein (which depends on the pH and the ionic strength) and the influence of the carrier on said parameters after contacting the carrier with said buffer containing solution. In accordance with the present invention it has been found that a suitable buffer is needed for the homogeneous distribution of the active agent onto the surface of the carrier, e.g. calcium phosphate, said buffer comprising preferably a weak acid, an alcohol and a saccharide. The solvent for the dissolution of the preferably bioresorbable polymer in which the protein or peptide is not soluble described by the method B of the present invention comprises a suitable organic solvent for the homogeneous distribution of the polymer onto the surface of the protein or peptide coated carrier e.g. dimethyl sulfoxide, anisol or glacial acid.

The term "thermal treatment" refers to a heating step which is applied after the solvent has been removed by drying to condense the polymeric phase by a definite collapse of the freeze dried structure and thus providing a dense and homogenous polymeric shell covering the ceramic surface of the granules. The purpose of this procedure is to modulate the release kinetics for the active substance and to achieve the desired mechanical properties and manifestation of the composite material. By variation of the process conditions during the annealing step, the mechanical properties and the manifestation of the implant material can be fine tuned.

In accordance with the present invention, the composite carrier is based on a calcium phosphate and a polymer, preferably a biodegradable polymer. In such a composite carrier the calcium phosphate shows excellent local buffering capacity and the permeable composite structure avoids even local pH decrease when the polymer is degraded in vivo. Cytotoxic side effects due to degradation of the polymer are, hence, reduced or avoided. This is especially valid, since the ceramic carrier is chief ingredient of the ceramic carrier/polymer composite carrier material of the presentinvention, which preferably contains less than 60% of the polymer, most preferably less than 50% of PLGA even more preferably equal or less than 40 % of PLGA.

In case of the calcium phosphate the ceramic carrier/polymer composite carrier material of the present invention preferably contains less than 80% of the calcium phosphate, most preferably less than 60% of calcium phosphate even more preferably equal or less than 50 % calcium phosphate.

In case of an additionally filler material e.g. Saccarides (Sucrose) Salts (NaCl) or PEG to enhance the porosity of the ceramic carrier/polymer composite carrier material of the present invention preferably contains less than 60% of the filler material, most preferably less than 50% of the filler material even more preferably equal or less than 45 % of filler material.

The temperature should be equal or higher than the glass transition temperature of the corresponding polymer system. For thermal sensitive active agents the glass transition temperature of the polymer can be decreased by the use of plasticizers, e.g. polyethylene glycol. The thermal treatment applies a temperature between the final drying temperature of ≥ 20°C, preferably ≥ 25°C, and most preferably ≥ 30 °C and the maximum temperature, limited by the active agent of ≤ 60 °C, preferably ≤ 55, and most preferably ≤ 50 °C. The time range for the thermal treatment in a preferred embodiment is as follows: Heating from 20 °C up to 60 °C in 30 minutes following by an isothermic period of 50 minutes at this temperature. Afterwards the samples are cooled down to 20 °C for 1 hour. The integrity of the active agent was determined after extraction the polymeric shell as demonstrated in example 4.

The invention encompasses a pharmaceutical composition comprising the material of the invention or a material, which is obtainable by the method of the invention.

The product of the present invention can be formulated as a pharmaceutical composition or a medical material. The composition of said product may comprise additional compounds like stabilizers, buffer substances and other excipients. The amount of the product of the present invention applied to the patient will be determined by the attending physician and other clinical factors; preferably in accordance with any of the above described methods. As it is well known in the medical arts, the amount applied to a patient depends upon many factors, including the patient's size, body surface area, age, sex, time and route of administration, general health conditions, and other drugs being administered concurrently. Progress can be monitored by periodic assessment.

Thanks to the present invention, it is possible to treat various bone defects including large cavities in a new manner. In particular, large cavities could not or only under use of autogenous bone material be efficiently treated. However, due to the reliable and efficient osteoinductive and the osteoconductive properties of the material of the present invention or the material which can be obtained by the method of the invention, treatment of bone defects which requires extensive bone augmentation or repair has now become possible without a second surgery for gaining autologous bone material.

The invention also encompasses the use of the material of the invention or a material which is obtainable by the method of the invention for the preparation of a pharmaceutical composition for bone augmentation.

The term "bone augmentation" refers to the induced formation of bone, which is indicated in order to treat bone defects, cavities in bones, or to treat diseases and disorders accompanied with loss of bone tissue or to prepare the subsequent setting of an implant. The diseases and disorders described in the following are well known in the art and are described in detail in standard medical text books such as Pschyrembel or Stedman.

Preferably, said bone augmentation follows traumatic, malignant or artificial defects.

Another embodiment of the present invention relates to the use of the material of the invention or the preparation of a pharmaceutical composition for treating bone defects.

More preferably, said bone defects are long bone defects or bone defects following apicoectomy, extirpation of cysts or tumors, tooth extraction, or surgical removal of retained teeth.

The invention also relates to the use of the material of the invention for filing of cavities and support guided tissue regeneration in periodontology.

Another embodiment of the present invention relates to the use of the material of the invention for the preparation of a pharmaceutical composition for sinus floor elevation, augmentation of the atrophied maxillary and mandibulary ridge and stabilization of immediate implants.

Also within the scope of the present invention is a method for treating one or more of the diseases referred to in accordance with the uses of the present invention, wherein said method comprises at least the step of administering the material of the invention in a pharmaceutically acceptable form to a subject. Preferably, said subject is a human.

Finally, the invention relates to a kit comprising the material of the invention.

The parts of the kit of the invention can be packaged individually in vials or other appropriate means depending on the respective ingredient or in combination in suitable containers or multicontainer units. Manufacture of the kit follows preferably standard procedures which are known to the person skilled in the art.

### Contribution to the field by the present invention

Polymer and carrier together form the ceramic/polymer composite carrier material of the present invention, which binds an osteoinductive active agent to result in the material of the present invention, in order to allow the sustained release of said active agent in vivo.

In accordance with the present invention, the composite carrier is based on a calcium phosphate and a polymer, preferably a biodegradable polymer. In such a composite carrier the calcium phosphate shows excellent local buffering capacity and the permeable composite structure avoids even local pH decrease when the polymer is degraded in vivo. Cytotoxic side effects due to degradation of the polymer are, hence, reduced or avoided. This is especially valid, since the ceramic carrier is chief ingredient of the ceramic carrier/polymer composite carrier material of the present invention, which preferably contains less than 20% of the polymer, most preferably less than 20% of PLGA.

Furthermore, the ceramic/polymer composite carrier material of the present invention shows improved mechanical stability compared to conventional systems using polymeric granules for retarded release. In one preferred embodiment, the resulting composite carrier material has mechanical properties, which exceed the mechanical properties of purely polymere based scaffolds. In porous embodiments the composite matrix allows for improved osteoconductive properties compared to prior art systems due to the porous system, in particular free of interconnected pores.

The ceramic/polymer composite carrier material of the present invention is suitable to replace conventional polymeric scaffolds important for retarded release. Due to the homogeneous coating of the system (ceramic carrier plus polymer coating), the amount of polymer can be significantly reduced compared with totally polymer based scaffolds, which reduced amount of polymer leads to a reduced risk of cytotoxicity. A further aspect of the invention is the increased mechanical stability of the composite material compared with totally polymer based scaffolds.

The invention will now be described by reference to the following examples which are merely illustrative and which shall not limit the scope of the present invention. Some of the measures and results of the methods set forth in the examples can be obtained from the accompanying figures.

### Examples

### Example 1: Solvent screening

Step 1: Coating of beta-TCP with rhGDF-5: 500 mg β-TCP (0.5 - 1.0 mm granule size) are placed in a dry form in a 6R-glass. The stock solution of rhGDF-5 (4 mg/ml in 10 mM HCl) is diluted to 1 g/ml (with the corresponding coating buffer). 425 µl of the rhGDF-5 solution obtained in that manner are pipetted on the beta-TCP and adsorbed. The damp granulate is incubated for 1 hour at 25°C and then lyophilised.
Step 2: In the subsequent step 425 µl of the following organic solvents were incubated with the coated granules from step 1 for 30 min at room temperature. After drying a sample was taken. The remaining samples were tempered at 60 °C for 1 h.
From each sample the rhGDF-5 was extracted according Example 4 to quantify the modified species by RP-HPLC.

### Example 2: Manufacturing of MD05 500 retard (with a protein).

Provide 0.5 g of β-TCP in a 6R glass vial, take 475 µl of the coating solution (0.525 mg/ ml rhGDF-5 in 10.0 mM acetic acid, 2.5 mM HCl, 10.0 % saccharide).
Dry by freeze-drying. Second coating by adding 425 µl of PLGA (Resomer RG 502 H) dissolved in DMSO, containing 150 mg PLGA/ ml DMSO.
Again drying by freeze-drying , including a terminal treatment step by 60 °C over 1 hour.

### Example 3: Release study of MD05 retard.

MD05 retard (containing a protein; rhGDF-5).
Weight 150 mg of MD05 retard into a 50 ml tube, ad 48 ml alpha-MEM-medium including 10 % of FCS and shake continuously at 4 °C and 37 °C for *defined* time, (final concentration of the release-assay is ~1.6 µg rhGDF-5/ ml medium). ***The defined time is up to 30 dayes*****....*****würde ich aber offen lassen je nach Wirkstoff.***

At defined time, an aliquot of 100 µl was taken (the taken volume will not be replaced), centrifuged for 5 minutes at 13 000 rpm, the supernatant is frozen at -70°C.
The quantification of the selected aliquots will be done by Elisa-assay.

MD05 retard (containing a peptide, Parathormon, PTH 1-34)
0.2 g of product is taken in 1 ml PBS (final concentration of PTH is 0.015 µg/ml)incubated at 4°C and 37 °C under continuously shaking.
At defined time, the incubation medium was replaced with fresh PBS.
The supernatant was centrifuged at 13000 rpm for about 5 minutes, an aliquot from 250 µl was analyzed by HPLC.

### Example 4: Quantification and determination of chemical modifications of the extracted polypeptide:

### Method A for bone growth factor

The amount of chemical modifications i.e. oxidation of bone growth factor in solutions containing extracted protein was determined by RP-HPLC. The sample is applied to a Vydak C8-18 column (2 x 250 mm) which has been equilibrated with 0.15 % TFA, 20 % acetonitrile. After washing of the column, the elution is performed with a mixture of 0.1 % TFA, 20 % acetonitrile, and a stepwise gradient of 20 % - 84 % acetonitrile (flow: 0.3 ml/min). The elution is observed by measuring the absorption at 215 nm. The quantification is calculated by the ratio of the peak area of modified species to the total peak area.

### Method B for peptide

The amount of chemical modifications, i.e. oxidation of bone growth factor in solutions containing extracted protein was determined by RP-HPLC. The sample is applied to a Vydak C8-18 column (4.6 x 250 mm) which has been equilibrated with 0.15 % TFA, 13.5 % acetonitrile. After washing of the column, the elution is performed with a mixture of 0.1 % TFA, 13.5 % acetonitrile and a stepwise gradient of 13.5 % - 84 % acetonitrile (flow: 0.5 ml/min). The elution is observed by measuring the absorption at 215 nm. The quantification is calculated by the ratio of the peak area of modified species to the total peak area.

### Example 5: Quantification of soluble aggregates in solutions containing extracted polypeptides (Proteins and peptides as well):

The amount of soluble aggregates in samples containing extracted protein was determined by size exclusion HPLC. The column (TSK 3000) was equilibrated with 50 mmol/l phosphoric acid, 50 mmol/l acetic acid/NaOH, pH 3.0.
The elution is observed by UV-detection at 220 nm. The quantification is calculated by the ratio of the aggregate peak area to the total peak area.

### Example 6: Stability testing of rhGDF-5 coated β-TCP granules in various organic solvents

The amount of solvent induced protein degradation was determined by incubating 500 mg of rhGDF-5 coated granules with 425 µl of the solvent for 30 min. Afterwards the solvent were removed by evaporation under vacuum and analyzed by RP-HPLC described in example 4 and 5.

### Example 7: Stability testing of rhGDF-5 coated β-TCP granules after drying from various organic solvents and annealing

The amount of solvent induced protein degradation during the annealing was determined by incubating 550 mg of rhGDF-5 coated granules with 425 µl of the solvent for 30 min. The solvent were removed by evaporation under vacuum and afterwards the vials were heated up at a temperature of 60 °C for 1 hour in an oven and analyzed by RP-HPLC described in example 4 and 5.

### Example 8: Stability testing of pure rhGDF-5 after drying from various organic solvents and annealing

To analyze the effect of various organic solvents on the pure GDF-5 100 µg GDF-5 were dryed under reduced pressure and 100µl of the solvent were added. The samples were incubated for 1 hour and dried again. The GDF-5 were dissolved in extraction buffer and analyzed by RP-HPLC described in example 5.

Example 9: Stability testing of rhGDF-5 coated β-TCP granules after drying from various organic solvents and annealing with optimized lyophilization conditions.
To measure the effect of the optimized manufacturing process on the solvent induced GDF-5 degradation 425 µl of the organic solvents were added to 550 mg MD05 incubated for 30 minutes and dried under the optimized lyophilization conditions described in Fig 10. The GDF-5 degradation during manufacturing were quantified by RP-HPLC described in example 4 and 5.

Example 10: Stability testing of rhGDF-5 coated β-TCP granules with PLGA shell after drying from various organic solvents and annealing with optimized conditions. 425 µl of a PLGA solution in DMSO (5 % w/v and 20 % w/v) were added to 550 mg of rhGDF-5 coated granules and dried under the optimized lyophilization conditions described in Fig 10. The GDF-5 degradation during this process was quantified by RP-HPLC described in example 4 and 5.

### References:

Agrawal, C. M. et al. (1997) "Technique to Control pH in Vicinity of Biodegrading PLA-PGA Implants"; J. Biomed. Mater. Res. (Appl. Biomater.), 38: 105-114

Breitenbach Jörg, (2002) "Melt extrusion: from process to drug delivery technology", Eur. J. Pharm. Biopharm. 54, 107-117

Celeste A.J., et al. (1990) "Identification of transforming growth factor ..."; Proc. Natl. Acad. Sci. U.S.A. 87:9843-9847.

Chang, S. et al. (1994); "Cartilage-derived morphogenetic proteins..."; J. Biol. Chem. 269: 28227-28234.

Draenert, K. et al (2001), Trauma Berufskrankh. 3, 293 - 300.

Driessens, F.C.M. et al. (2002) "The Ca/P range of nanoapatitic calcium phosphate cements" Biomaterials 23, 4011-4017

Dunn et al. US patent 5,702,716

Durucan, C. et al.; "Calcium-deficient hydroxyapatite-PLGA composites: mechanical and microstructural investigation." J. Biomed. Mater. Res. 51:726-724.

EMEA, ICH Topic Q 3C, Impurities: Residual Solvents

Friess, W. et al. (1998); Pharm. Dev. Technol. 4, 387 - 396.

Friess, W. (1999); "Collagen - biomaterial for drug delivery"; Eur J Pharm Biopharm 45: 113-36.

Gao, T et al. (1996); Int. Orthopaedics 20, 321 - 325.

Gombotz, W et al. (1996) in Formulation, characterization and stability of protein drugs, Plenum Press, New York, USA, pp 219 - 245.

Griffith, D.L. et al. (1996); "Three-dimensional structure of recombinant human..."; Proc. Nati. Acad. Sci. U.S.A. 93: 878-883.

Herbert, P. et al., 1998. A large scale process to produce microencapsulated Proteins. Pharm Res., 15: 357-361
Hoetten, G. et al. (1994); "Coning and expression of recombinant human growth/differentiation factor 5."; Biochem. Biophys. Res. Commun. 204: 646-652.

J.O. Hollinger et al. (1996); "Poly(α-hydroxy acids): carriers for bone morphogenetic proteins, Biomaterials"; 17: 187-194

Hotz, G et al. (1994); Int. J. Oral Maxillofac. Surg. 23, 413 - 417.

Katagiri, T. et al. (1990); Biochem. Biophys. Res. Commun. 172: 295-299.

Kenley, R.A., et al. (1993) Pharm. Res. , 10 (10) 1393-1401.

Lind, M et al. (1996); J. Orthopaedic Res. 14, 343 - 350

Lind, M. (1996); Acta. Orthop. Scand. 67: 407-17.

Middleton, J.C. et al. (2000); "Synthetic biopolymers as orthopedic devices"; Biomaterials 21: 2335-2346

Nishitoh, H. et al (1996); J Biol. Chem. 271: 21345-21352.

Oezkayanak, E. et al. (1990); "OP-1 cDNA encodes an osteogenic protein in the TGF-beta family"; EMBO J. 9: 2085-2093

Rueger, J.M. et al (1996); "Knochenersatzmittel"; Unfallchir. 99: 228-236

Scheufler, C. et al. (1990); "Crystal structure of human bone morphogenetic protein-2 at 2.7 A resolution".

Schiller, C. et al. (2003); "Carbonated calcium phosphates are suitable pH-stabilising fillers for biodegradable polyesters"; Biomaterials 24:2037-2043.

Schmidmaier, G. et al. (2000); "Local liberation of IGF-I and TGF-beta 1 from a biodegradable poly(D,L-lactide) coating of implants accelerates fracture healing" Chirurg 71: 1016-1022

Schmitt, J. et al. (1999); J. Orthop. Res. 17: 269-278.

Seeherman, H. (2003), "A review of preclinical program development for evaluating in injectable carriers for osteogenic factors" J. Bone. Joint. Surg. Am. 85-A Suppl. 3, 96-108

Shively M.L. et al. (1995), "Physico-chemical characterization of a polymeric injectable implant delivery system" J. Controlled Rel. 33, 237-243

Shore, E.M. et al. (1997); "Human bone morphogenetic protein-2 (BMP-2) genomic DNA sequence".

Storm & Kingsley (1999); Development Biology, 209, 11-27.

Terheyden, H et al. (1997); Mund Kiefer Gesichtschir. 1, 272 - 275.

Tormälä, P et al. (1995) "Biodegradable polymers in orthopaedics: experimental and clinical" Mat. Clin. Appl. 639-651

Tormälä, P et al. (1992) "Biodegradable self-reinforced composite materials; manufacturing structure and mechanical properties" Clin. Mater. 10, 29-34

Vert, M. (1989); "Bioresorbable polymers for temporary therapeutic applications" Angew. Makromol. Chem. 166/167: 155-168

Wang, E.A. et al. (1990); "Identification of transforming growth factor beta family members present in bone-inductive protein purified from bovine bone."; Proc. Natl. Acad. Sci. U.S.A. 87: 9843-9847.

Wintermantel, E. et al., Medizintechnik mit biokompatiblen Werkstoffen und Verfahren, S. 7, 3. Auflage, Springer 2002, ISBN 3-540-41261-1

Wozney, J.M. et al. (1998); Clin. Orthop. 346: 26-37.

Wozney, J.M. et al. (1988); Science 242: 1528-1534.

## Claims

1. A method for the production of a material comprising the steps of:
(a) providing a solution comprising an active agent, and a polymer, wherein said active agent and said polymer are kept dissolved for a time sufficient to allow homogenous coating of a carrier, preferably a ceramic carrier, when said carrier is contacted with said solution;
(b) contacting the solution of step (a) with a carrier, preferably a ceramic carrier, more preferably a ceramic carrier containing calcium phosphate
(c) allowing homogenous coating of the surface of said carrier with said dissolved active agent and polymer;
(d) drying the coated carrier obtained in step (c); and
(e) thermally treating said coated carrier obtained in step (d).

2. A method for the production of a material comprising the steps of:
(a) providing an aqueous solution comprising an active agent and a buffer, which buffer keeps said active agent dissolved for a time sufficient to allow homogenous coating of a carrier, preferably a ceramic carrier when said carrier is contacted wih said solution;
(b) contacting the solution of step (a) with a carrier, preferably a ceramic carrier, more preferably a ceramic carrier containing calcium phosphate;
(c) allowing homogenous coating of the surface of said carrier with said dissolved active agent;
(d) drying the coated carrier obtained in step (c);
(e) providing a further solution comprising dissolved polymer, which polymer stays dissolved for a time sufficient to allow homogenous coating of the carrier obtained in step (d) when said carrier is contacted with said solution, wherein the active agent coated onto said carrier is not soluble in said solution;
(f) drying the polymer coated carrier obtained in step (e); and
(g) thermally treating said coated carrier obtained in step (f).

3. The method of claim 1 or 2, wherein said carrier is in a granular form, preferably in the form of free flowing granules.

4. The method of claim 3, wherein said material is formed to exhibit a solid three dimensional scaffold, preferably with the manifestation of a load bearing three-dimensional implant with mechanical properties preferably similar to bone, the method comprising the step of:
(x) annealing said granules

5. The method of any of claims 1 to 4, wherein said carrier contains a calcium phosphate selected from beta tricalcium phosphate, alpha tricalcium phosphate, apatite and a calcium phosphate containing cement.

6. The method of any one of claims 1 to 5, wherein said material has osteoinductive and osteoconductive properties *in vivo*.

7. The method of any one of claims 1 to 6, wherein said polymer is biocompatible, biodegradable and/or bioresorbable.

8. The method of any one of claims 1 to 7, wherein said biocompatible, biodegradable, and/or bioresorbable polymer is selected from a poly(α-hydroxy acids), poly (ortho esters), poly(anhydrides), poly(aminoacids), polyglycolids (PGA), polylactids (PLLA), poly(D,L-lactide) (PDLLA), poly(D,L-lactide co-glycolide) PLGA), poly(3-hydroxybutyricacid) (P(3-HB)), poly(3-hydroxy valeric acid) (P(3-HV)), poly(p-dioxanone) (PDS), poly(ε-caprolactone) (PCL), polyanhydride (PA), polyorthoester, polyethylene (PE), polypropylene (PP), polyethyleneterephthalate (PET), polyglactine, polyamide (PA), polymethylmethacrylate (PMMA), polyhydroxymethylmethacrylate (PHEMA), polyvinylchloride (PVC), polyvinylalcohole (PVA), polytetrafluorethylene (PTFE), polyetheretherketone (PEEK), polysulfon (PSU), polyethyleneglycol (PEG), polyvinylpyrolidone, polyurethane or polysiloxane.

9. The method of any one of claims 1 to 8, wherein said active agent is an osteoinductive polypeptide (protein or peptide).

10. The method of claim 9, wherein said osteoinductive polypeptide is a member of the TGF-beta family, preferably a member of the BMP subfamily.

11. The method of claim 10, wherein said member of the BMP family is selected from the group consisting of BMP-1, BMP2, BMP-3, BMP-4, BMP-5, BMP-6, BMP7, BMP-8, BMP-9, BMP-10, BMP-11, BMP-12, BMP-13, BMP-14, BMP-15 and BMP-16.

12. The method of claim 10, wherein said member of the TGF-β family is selected from the group consisting of GDF-1, GDF-2, GDF-3, GDF-4, GDF-5, GDF-6, GDF-7, GDF-8, GDF-9, GDF-10 and GDF-11.

13. The method of any one of claims 1 to 8, wherein said active agent is selected from the group consisting of hormones, cytokines, growth factors, antibiotics, steruoids, prostaglandines and other natural or synthesised drug substances.

14. The method of claim 13, wherein said active agent is parathyroid hormone (PTH) and/or PTH 1-34 peptide.

15. The method of any one of claims 1 to 14, wherein said solution in step 1(a) and step 2(e) contains as solvent a compound selected from anisole, tetramethylurea, acetic acid, dimethylsulfoxide and tert-butanol, acetone, 1-butanol, 2-butanole, butyl acetate, tert-butylmethyl ether, cumene, ethanol, ethyl acetate, dieethylether, ethylformate, formic acid, isobutyl acetate, isopropylacetate, methyl acetate, 3-methyl-1-butanol, methylethyl ketone, methylisobutylketone, 2-methyl-1-propanole, pentane, 1-pentanol, 2-propanol, propylacetate, and tetrahydrofurane.

16. The method of any one of claims 1 to 15, wherein said prepared material is free of toxic substances.

17. The method of any one of claims 1 to 15, wherein said prepared material is free of infectious material.

18. A material which is obtainable by the method of any one of claims 1 to 17.

19. A pharmaceutical composition comprising the material of claim 18.

20. Use of the material of claim 18 for the preparation of a pharmaceutical composition for bone augmentation.

21. The use of claim 20, wherein said bone augmentation follows traumatic, malignant or artificial defects or is a prerequisite for the subsequent setting of an implant.

22. Use of the material of claim 18 for the preparation of a pharmaceutical composition for treating bone defects.

23. The use of claim 22, wherein said bone defects are long bone defects, defects in the maxillofacial area or bone defects following apicoectomy, extirpation of cysts or tumors, tooth extraction, or surgical removal of retained teeth.

24. Use of the material of claim 18 for the preparation of a pharmaceutical composition for treating degenerative and traumatic disc disease.

25. Use of the material of claim 18 for the preparation of a pharmaceutical composition for treating bone dehiscence.

26. Use of the material of claim 18 for the preparation of a pharmaceutical composition for sinus floor elevation or augmentation of the atrophied maxillary or mandibular ridge.

27. Use of the material of claim 18 for the preparation of a pharmaceutical composition for filling cavities and/or support guided tissue regeneration in periodontology.

28. A kit comprising the material of claim 18.
